# EUROPEAN PATENT SPECIFICATION

(11) **EP 2 394 621 B1**
(45) Date of publication and mention of the grant of the patent: **14.10.2015**
(21) Application number: 10738552.8
(22) Date of filing: 03.02.2010
(51) Int. Cl.: A61F 13/15, A61F 13/472, A61F 13/47

(54) **DISPOSABLE BODILY FLUENT ABSORBENT WEARING ARTICLE**
EINWEG-KLEIDUNGSSTÜCK ZUR AUFNAHME VON KÖRPERFLÜSSIGKEITEN
ARTICLE À PORTER JETABLE ABSORBANT UN LIQUIDE CORPOREL

(30) Priority: 06.02.2009 JP 2009026719
(43) Date of publication of application: 14.12.2011
(73) Proprietor: Uni-charm Corporation, Ehime 799-0111 (JP)
(72) Inventor: SASAYAMA, Kenichi, Kanonji-shi Kagawa 769-1602 (JP); KONISHI, Takayoshi, Kanonji-shi Kagawa 769-1602 (JP)
(74) Representative: Eke, Philippa Dianne
(86) International application number: PCT/JP2010/051513
(87) International publication number: WO 2010/090221

(56) References cited:
- EP-A1- 0 888 764
- EP-A1- 1 407 739
- EP-A1- 1 424 051
- EP-A2- 0 302 523
- JP-A- 7 047 096
- JP-A- 2007 082 578
- JP-A- 2007 111 194
- JP-A- 2008 073 356
- US-A- 4 595 392
- US-B1- 6 680 421

## Description

### {Technical Field}

The present invention relates to disposable bodily fluid absorbent wearing articles suitable to be put on a wearer's crotch skin and, more particularly, to disposable bodily fluid absorbent wearing articles suitable for use as hemorrhoid pads, labial pads and the like.

### {Background}

Conventionally, disposable wearing articles adapted to be aligned with and put against a target portion in the crotch region of a wearer, such as the female genital organs, to absorb bodily fluids are known. Articles of such kind particularly being formed so that the midsection in the width direction becomes higher than the peripheral region are also known.

For example, JP 1999-313851 A1 (PTL 1) discloses a sanitary napkin having an inverted V-shaped cross section taken in a width direction. This sanitary napkin has a lower surface of a flat backsheet member being coated with pressure-sensitive adhesives and extending outward in the width direction beyond opposite side edges of an inverted V-shaped core. In use, this sanitary napkin is fastened to the wearer's undergarment with the pressure-sensitive adhesives.

JP 2007-111191 A1 (PTL 2) discloses a labial pad having an inverted V-shaped cross section taken in a width direction. In this pad, a connector member made of elastic material extends in the width direction and is bonded to flaps defined on the outside of transversely opposite side edges of an inverted V-shaped absorbent core. JP 7 047096 (PTL 3) also describes a labial pad, and EP 0 302 523 (PTL 4) discloses a pad for absorption of human exudate.

### {Citation List}

### {Patent Literature}

{PTL 1} JP 1999-313851 A1
{PTL 2} JP 2007-111191 A1
{PTL 3} JP 7 047096 A
{PTL 4} EP 0 302 523 A2

### {Summary}

### {Technical Problem}

For these wearing articles of the known art, having the inverted V-shaped cross section taken in the width direction, it is preferable, in actual use, to fit the apex of the inverted V-shaped cross section accurately to the target region of the wearer's crotch. However, in the sanitary napkin disclosed by PTL 1, once the release paper covering the pressure-sensitive adhesives has been peeled off, the pressure-sensitive adhesives come closest to the inner surface of the wearer's undergarment and readily come in contact with the undergarment. After the pressure-sensitive adhesives have been put in contact with the inner surface of the undergarment, it is difficult to adjust the position of the sanitary napkin relative to the inner surface of the undergarment.

The labial pad disclosed by PTL 2 claims that, once the pad has been put between vaginal lips, the connector member made of an elastic material reliably prevents the pad from being displaced out of alignment with the vaginal lips under the repelling force of the connector means. However, the stability of the pad between the vaginal lips largely depends on the initial positioning of the pad and the pad is likely to be readily displaced from the vaginal lips.

An object of the present invention is to improve a disposable bodily fluid absorbent wearing article so that the article can be easily fitted to a desired region of the wearer's body.

### {Solution to Problem}

The present invention therefore provides the disposable bodily fluid absorbent wearing article of independent Claim 1. The dependent claims specify preferred but optional features. According to the present invention, there is provided a disposable bodily fluid absorbent wearing article having a longitudinal direction, a transverse direction and a thickness direction being orthogonal one to another, and comprising a liquid pervious skin-contactable sheet, a garment-contactable sheet and a bodily fluid absorbent core sandwiched between the skin-contactable sheet and the garment-contactable sheet in the thickness direction so as to form a flat pad being symmetric about a center line extending in the longitudinal direction and bisecting a dimension of the pad in the transverse direction wherein the flat pad is bent in two halves along the center line with the skin-contactable sheet facing outward so as to define an inverted V-shaped cross section.

The improvement according to the present invention is characterized as follows: the respective halves of the garment-contactable sheet facing each other in the transverse direction about the center line are partially connected to each other by connector means so that the pad may be maintained in the state of being bent in the inverted V-shape, and the bodily fluid absorbent wearing article is provided with fastening means adapted for detachably fastening the garment-contactable sheet to the wearer's garment.

The connector means comprises a connector sheet having both lateral edge portions in the transverse direction attached to portions of the respective halves of the garment-contactable sheet.

The invention includes an embodiment wherein the skin-contactable sheet and the garment-contactable sheet extend outward beyond a peripheral edge of the core and are layered and bonded together along respective extending portions to define a peripheral region of the bodily fluid absorbent wearing article, and the fastening means are formed inside the peripheral region of the bodily fluid absorbent wearing article.

The invention includes an embodiment wherein the flat pad has a contour line defining a planar shape thereof and the contour line includes convexly curved segments so as to be furthest from the center line and the flat pad is formed with the fastening means inside the convexly curved segments.

The invention includes an embodiment wherein the fastening means are formed of one of a pressure-sensitive adhesive and a hook member of a mechanical fastener.

The invention includes an embodiment wherein the skin-contactable sheet, the garment-contactable sheet and the connector sheet are respectively formed of water-disintegrable sheets and the core is a mass of liquid-absorbent fibers each having a fiber length of 20mm or less.

The invention includes an embodiment wherein any one of the skin-contactable sheet, the garment-contactable sheet and the connector sheet is formed of a water-disintegrable nonwoven fabric containing ultrafine thermoplastic fibers each having a fineness in a range of 0.01 to 0.5dtex and a fiber length in a range of 3 to 10mm in an amount of 10 to 50% by mass.

### {Advantageous Effects of Invention}

In the disposable bodily fluid absorbent wearing article according to the present invention, the connector sheet attached thereto is provided with the fastening means adapted for detachably fastening the bodily fluid absorbent wearing article to the wearer's garment. With such an arrangement, when this article is used for a hemorrhoidal patient in close alignment with the anus, for example, the article can be fastened to the patient's garment such as pants with this fastening means. In this way, the article should not be displaced out of alignment with the anus even when the patient moves his or her body.

### {Brief Description of Drawings}

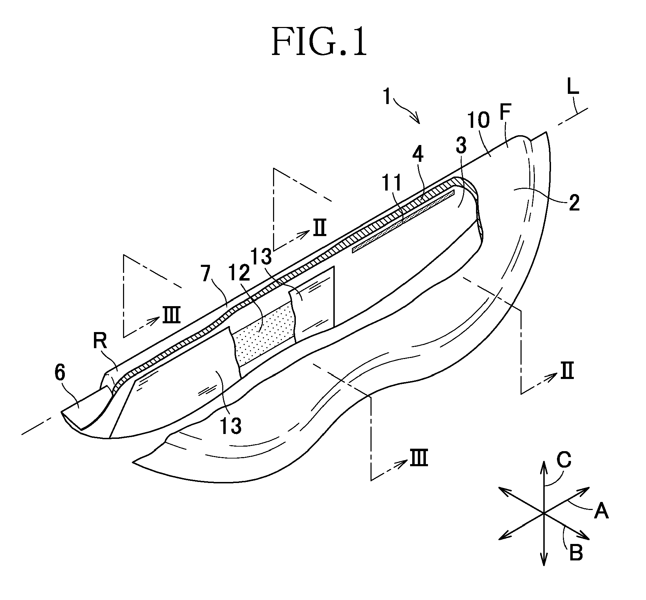

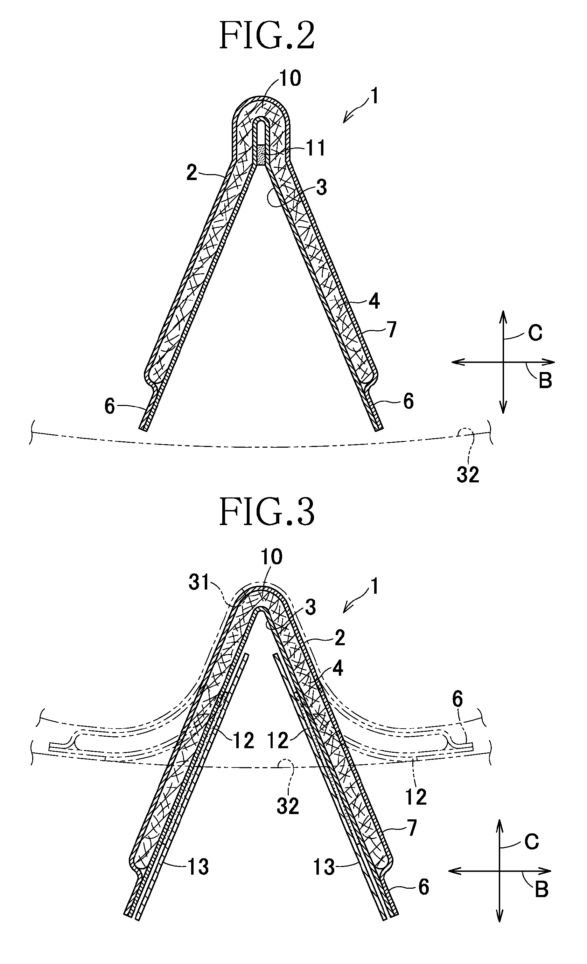

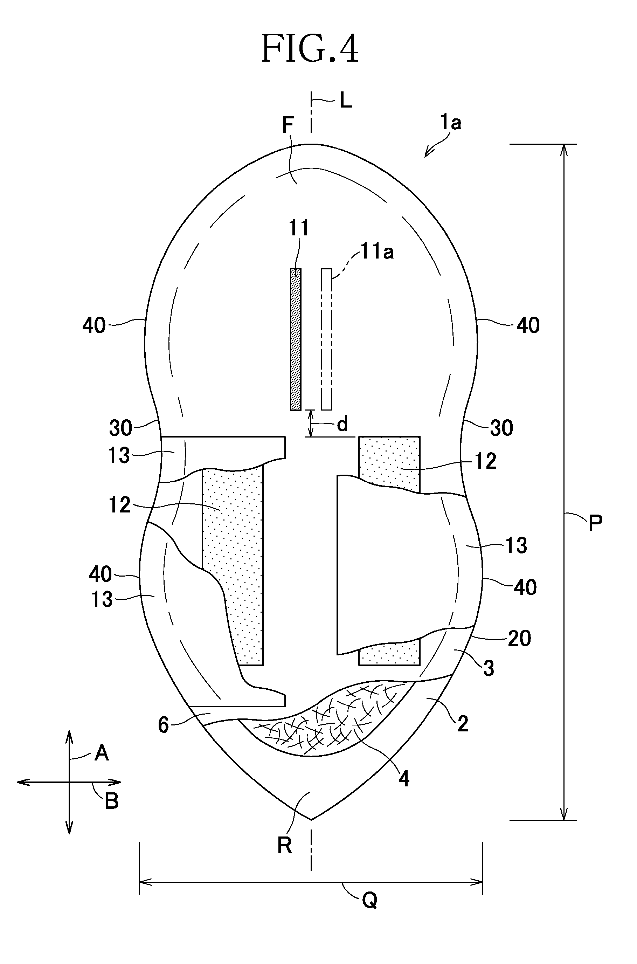

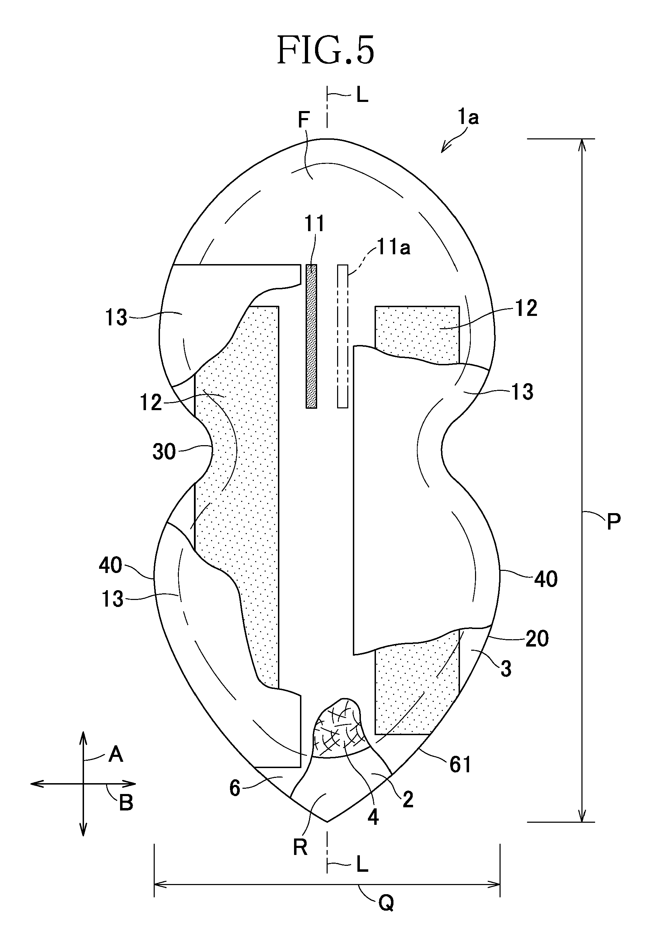

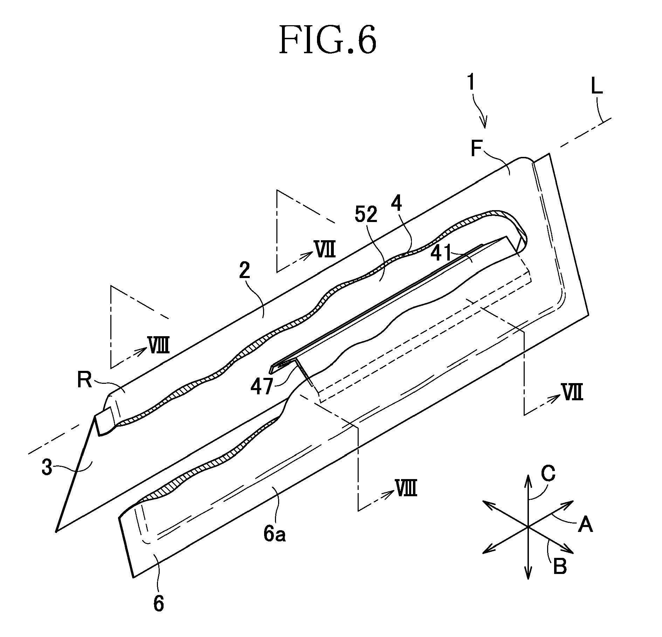

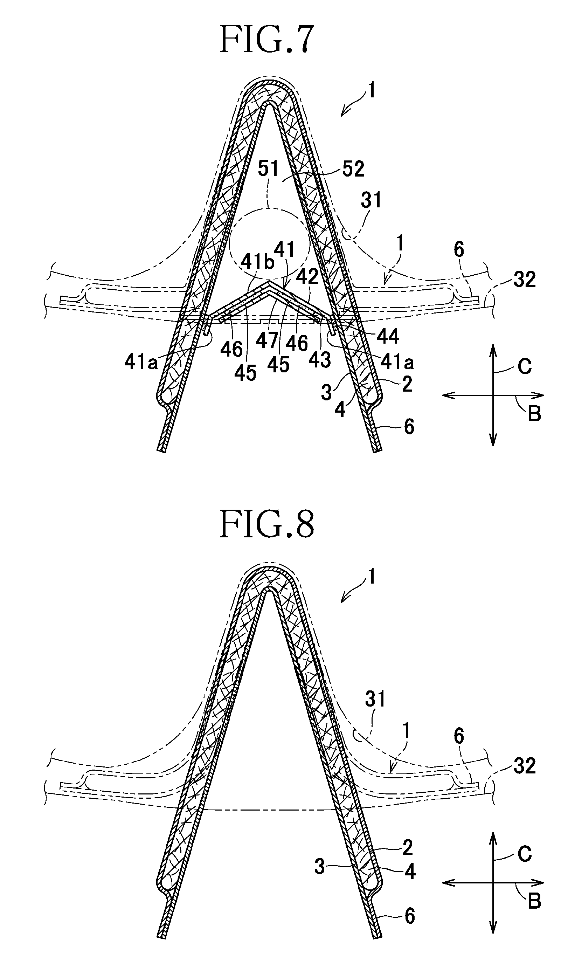

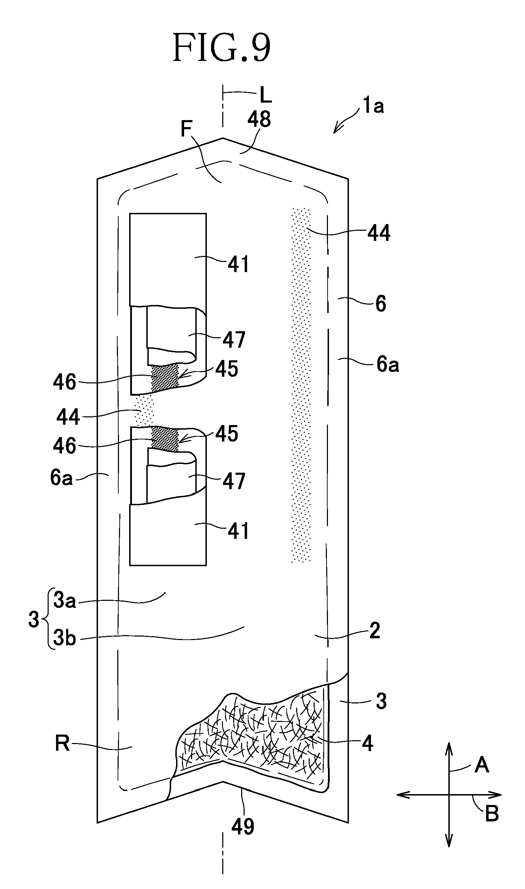

{Fig. 1} Partially cutaway perspective view of a disposable bodily fluid absorbent wearing article (hemorrhoid pad), being a reference example.
{Fig. 2} Sectional view taken along line II-II in Fig. 1.
{Fig. 3} Sectional view taken along line III-III in Fig. 1.
{Fig. 4} Partially cutaway plan view showing the bodily fluid absorbent wearing article in Fig. 1 as has been flatly unfolded.
{Fig. 5} View similar to Fig. 4, showing another reference example.
{Fig. 6} View similar to Fig. 1, showing an embodiment of the invention.
{Fig. 7} Sectional view taken along line VII-VII in Fig. 6.
{Fig. 8} Sectional view taken along line VIII-VIII in Fig. 6.
{Fig. 9} Partially cutaway plan view showing the bodily fluid absorbent wearing article in Fig. 6 as has been flatly unfolded.

### {Description of Embodiments and Reference Examples}

Details of a disposable bodily fluid absorbent wearing article according to the invention will be more fully understood from the description given hereunder with reference to the accompanying drawings.

Figs. 1, 2 and 3 are, respectively, the partially cutaway perspective view of a hemorrhoid pad 1 as a reference example of the disposable bodily fluid absorbent wearing article, the sectional view taken along line II-II in Fig. 1 and the sectional view taken along line III-III in Fig. 1. The pad 1 has a longitudinal direction A, a transverse direction B and a height direction C being orthogonal one to another and includes a front end portion F and a rear end portion R opposed to each other in the longitudinal direction A. The pad 1 is shaped symmetrically about a center line L bisecting a dimension in the transverse direction B. Referring to the sectional views of Figs. 2 and 3, the pad 1 is bent in two along the center line L so as to define an inverted V-shape having an apex at the highest level in the height direction C. Referring to Fig. 1, the apex 10 comes into line with the center line L and extends in the longitudinal direction A.

The pad 1 includes a skin-contactable sheet 2, a garment-contactable sheet 3 and a bodily fluid absorbent core 4 sandwiched between these two sheets 2, 3. The skin-contactable sheet 2 is liquid-pervious and the garment-contactable sheet 3 may be liquid-impervious or liquid-pervious, preferably, liquid-impervious. Both the skin-contactable sheet 2 and the garment-contactable sheet 3 extend outward beyond a peripheral edge of the core 4 and are layered and bonded together along respective extending portions with adhesives or by fusing thermoplastic synthetic resins contained in these sheets 2, 3 to define a peripheral region 6 of the pad 1. In the preferred peripheral region 6, the skin-contactable sheet 2 and the garment-contactable sheet 3 are bonded to each other particularly with water-soluble adhesives such as starch pastes. The inner side area surrounded by the peripheral region 6 defines a bodily fluid absorbent region 7.

In the inverted V-shaped section shown in Figs. 2 and 3 to be taken in the transverse direction B, the skin-contactable sheet 2 defines an outer surface of the pad 1 and the garment-contactable sheet 3 defines an inner surface of the pad 1. In Fig. 2 showing the section of the pad 1 taken in the front end portion F of the pad 1, respective halves of the garment-contactable sheet 3 facing each other about the center line L (See Fig. 1) are bonded to each other in the vicinity of the apex 10 with adhesives 11. In Fig. 3 showing the section of the pad 1 taken in the rear end portion R of the pad 1, the respective halves of the garment-contactable sheet 3 facing each other about the center line L are coated with pressure-sensitive adhesives 12 which are protectively covered with release sheets but not bonded to each other with the adhesives 11 as in the case of Fig. 2.

Fig. 4 is a partially cutaway plan view showing the pad 1 flatly unfolded after the respective halves of the garment-contactable sheet 3 bonded together with the adhesives 11 as seen in Fig. 2 have been peeled off from each other. While such flatly unfolded pad 1a is symmetric about the center line L, such symmetric relationship is not applicable to the adhesives 11. Specifically, the adhesives 11 are shown on one side of the center line L and the region to be bonded to the counterpart with the adhesives 11 are indicated on the other side of the center line L by an imaginary line 11a. The adhesives 11 are applied to the garment-contactable sheet 3 only on the side of the front end portion F, and most of the pressure-sensitive adhesives 12 are applied to the garment-contactable sheet 3 on the side of the rear end portion R opposite to the front end portion F. The adhesives 11 and the pressure-sensitive adhesives 12 are spaced at a distance d from each other in the longitudinal direction A. While a planar shape of the flat pad 1a is not specified according to the present invention, a contour line 20 defining the planar shape of the flat pad 1a curves alternately convexly and concavely on each side of the center line L according to the illustrated reference example, so that convex curve lines are formed on a front end 21 and a rear end 22 of the flat pad 1a. Respective segments of the contour line 20 defined on both sides of the center line L include concavely curved segments 30 and convexly curved segments 40 at the furthest distance from the center line L. In the flat pad 1a as illustrated by way of example, the pressure-sensitive adhesives 12 are applied in zones defined inside the respective convexly curved segments 40 inwards of the peripheral region 6. The longitudinal direction A, the transverse direction B and the center line L of the flat pad 1a formed in this manner correspond to the longitudinal direction A, the transverse direction B and the center line L in Fig. 1, respectively. The height direction C in Fig. 1 corresponds to the thickness direction (not shown) of the flat pad 1a in Fig. 4. The thickness direction of this flat pad 1a is orthogonal to the longitudinal direction A and the transverse direction B in Fig. 4. While a dimension P in the longitudinal direction A and a dimension Q in the transverse direction B of the flat pad 1a may be selected depending on the physical size of the wearer, a commonly used hemorrhoid pad 1 has the dimension P in a range of 8 to 15cm and the dimension Q in a range of 4 to 9cm.

The pad 1 may be put on the wearer's body, for example, in a sequence as follows: the pad 1 as a whole is inserted into bottom cleavage 31 with the apex 10 in the front end portion F aligned with and put against the anus of the hemorrhoidal patient, as indicated by imaginary lines in Figs. 2 and 3. In the course of insertion of the pad 1 in this manner, a finger may be inserted into a space defined between the respective halves of the garment-contactable sheet 3, 3 facing each other so as to define the inverted V-shape in the rear end portion R of the pad 1 to facilitate the operation of insertion. The release sheet 13 may be peeled off before the pad 1 is inserted into the bottom cleavage 31 or after the pad 1 has been aligned with and put against the anus of the wearer. With the pad 1 put against the anus, pants 32 into which the wearer has already stepped may be fully pulled up to put the inner surface of the pants 32 in close contact with the pressure-sensitive adhesives 12 and thereby to fasten the pad 1 to the inner surface. Thereupon, a region of the pad 1 including the pressure-sensitive adhesives 12 is deformed along the inner surface of the pants 32. While the rear end portion R of the pad 1 is deformed in this manner, the front end portion F not fastened to the pants 32 is kept in its inverted V-shape, i.e., its angular shape and apt to be biased in the opposite direction of the rear end portion R. Specifically, the front end portion F is apt to be raised from the inner surface of the pants 32 toward the anus, restricting the possibility that the pad 1 might come off from the bottom cleavage. Such behavior of the front end portion F advantageously functions to accelerate absorption of bodily fluids. In such a position of the front end portion F, the pants 32 and the peripheral region 6 are spaced from each other in the height direction C as indicated by imaginary lines in Fig. 2.

Such a manner of wearing the pad 1 ensures that the pressure-sensitive adhesives 12 applied to the pad 1 inside the peripheral region 6 are kept out of contact with the inner surface of the pants 32 regardless of the time point at which the release sheet 13 is peeled off and unless the inner surface of the pants 32 is pressed against the pressure-sensitive adhesives 12. More specifically, the pressure-sensitive adhesives 12 should not come in contact with the inner surface of the pants 32 in the course of pulling up of the pants 32. The pad 1 can be fastened to the inner surface of the pants 32 at the appropriate position thereof without any difficulty. After the pad 1 has been fastened to the inner surface of the pants 32 with the pressure-sensitive adhesives 12, the pad 1 should not be readily displaced out of alignment with the wearer's anus (not shown) even when he or she moves. Tack strength of the pressure-sensitive adhesives 12 is adjusted so that the pad 1 may be easily peeled off from the inner surface of the pants 32 and, in view of this, the pressure-sensitive adhesives 12 may be preferably selected from a group of water-soluble products such as a pressure-sensitive adhesives made of polyvinyl alcohol.

The pad 1 may be put on the wearer's body in an alternative sequence as follows: The pad 1 is fastened to the inner surface of the pants 32 at the appropriate position before the pad 1 is aligned with and put against the wearer's anus. The shape of the pad 1 in this step is indicated by imaginary lines in Fig. 3. The front end portion F of the pad 1 has its apex 10 maintained convex under the effect of the adhesives 11 and therefore the front end portion F is smoothly moved into the wearer's bottom cleavage 31 as the pants worn in a halfway manner are pulled up. For a patient regularly using the pad 1, the target region in which the pad 1 should be fastened to the pants 32 becomes constant and it is easy for such a wearer to know where such target region is.

In the pad 1, the skin-contactable sheet 2 may be formed, for example, by a liquid-pervious nonwoven fabric or woven fabric, or a perforated plastic film. The garment-contactable sheet 3 may be formed, for example, from liquid-pervious or liquid-impervious nonwoven fabrics or woven fabrics, or plastic films. However, liquid-impervious materials are preferably used in order to prevent any quantity of bodily fluids, once having been absorbed by the pad 1, from leaching out from the pad 1. The core 4 may be formed of fluff pulp or a mixture of the fluff pulp and super-absorbent polymer particles.

The skin-contactable sheet 2 and the garment-contactable sheet 3 may be formed of a water-disintegrable nonwoven fabric of which tissue in the form of a sheet is disintegrated into a plurality of fractions when this nonwoven fabric is stirred in a large amount of water. As an example of such nonwoven fabric, there are spun laced nonwoven fabrics including ultrafine thermoplastic synthetic fibers each having a fineness in a range of 0.01 to 0.5dtex and a fiber length in a range of 3 to 10mm in 10 to 50% by mass, chemical fibers each having a fineness in a range of 1 to 2dtex and a fiber length in a range of 5 to 20mm and/or pulp fibers having a freeness in a range of 600 to 770cc in 90 to 50% by mass. More specifically, there is a spun laced nonwoven fabric having a basis mass of 35g/m² and a thickness of 0.24mm including 0.3dtex ultrafine thermoplastic synthetic fibers in 20% by mass which are obtained by splitting a polyethylene terephthalate/nylon conjugate fibers each having a fineness of 3.3dtex and a fiber length of 5mm and being formed to be splittable into 11 fibers, and rayon fibers having a fineness of 1.1dtex and a fiber length of 7mm in 80% by mass. The spun laced nonwoven fabric is water-disintegrable and has a sufficient tensile strength, flexibility and liquid-perviousness to be used as the skin-contactable sheet 2. This nonwoven fabric may be coated with a water-soluble binder such as AQ55S produced by Eastman Chemical Company in 8% by mass of a total mass of the nonwoven fabric to obtain the nonwoven fabric which is water-disintegrable but sufficiently low in liquid-perviousness to be used as the garment-contactable sheet 3. In the pad 1 using the water-disintegrable nonwoven fabric, the core 4 is preferably formed of liquid-absorbent fibers such as pulp fibers having a fiber length of 20mm or less including none of super-absorbent polymer particles so that the core 4 also may be disintegrated into fractions when stirred in a large amount of water. This is because the super-absorbent polymer particles contained in the core 4 may form a water-insoluble block as the core 4 absorbs liquid.

While either water-soluble adhesives or hot melt adhesives may be used as the adhesives 11, it is preferred to use water-soluble adhesives from the viewpoint of improvement in the water-disintegration of the pad 1. If the garment-contactable sheet 3 of the pad 1 contains thermoplastic resin, the respective halves of the garment-contactable sheet 3 bent in the inverted V-shape may be locally fusion-bonded instead of bonding with the adhesives 11. In either case, it is preferable to constrict the area in which the respective halves of the garment-contactable sheet 3 are locally bonded together in order to prevent the water-disintegrable property of this sheet 3 from deteriorating.

Fig. 5 is a view similar to Fig. 4, showing another reference example. The flatly unfolded pad 1a according to this reference example is distinguished from the flatly unfolded pad 1a in Fig. 4 not only in its planar shape but also in the location of the pressure-sensitive adhesives 12. Specifically, the pressure-sensitive adhesives 12 extend into the front endportion F so as to be partially alongside the adhesives 11. It is possible to extend the range of coating with the adhesives 12 in such a manner. In this reference example, the pressure-sensitive adhesives 12 are applied to the inner surface of the garment-contactable sheet 3 inside the convexly curved segments 40 of the contour line 20 but with the outermost edge of the pressure-sensitive adhesives 12 coming into line with the contour line 20 along the concavely curved segments and in regions 61 of the rear end portion R defined in the vicinity of the center line L. In principle, the pressure-sensitive adhesives 12 should be applied to the garment-contactable sheet 3 inside the convexly curved segments 40 of the contour line 20. However, the pressure-sensitive adhesives 12 may be applied to the segments of the contour line 20 which will not come in contact with the inner surface of the pants 32 when the pad 1 is erected on the inner surface of the pants 32 in the inverted V-shape as viewed in Figs. 2 and 3. When the pad 1 corresponding to the flatly unfolded pad 1a of Fig. 5 is fastened to the inner surface of the pants 32, not only the rear end portion R but also the front end portion F can be fastened to the inner surface of the pants 32 and thereby it is assured to fasten the pad 1 to the pants 32 tightly. With regard to the pad 1 according to this reference example, also, the pressure-sensitive adhesives 12 should not come in contact with the inner surface of the pants 32 even if the release sheet 13 has been peeled off.

Figs. 6, 7 and 8 are views similar to Figs. 1, 2 and 3, respectively, showing an embodiment of the present invention wherein Fig. 6 is a partially cutaway perspective view showing the hemorrhoidal pad 1, Fig. 7 is a sectional view taken along line VII-VII in Fig. 6 and Fig. 8 is a sectional view taken along line VIII-VIII in Fig. 6. The pad 1 shown in Figs. 6 and 7 is distinguished from the pad 1 shown in Fig. 1 in that the respective halves of the garment-contactable sheet 3 in the front end portion F of the pad 1 facing each other in the transverse direction B about the center line L are connected with each other by means of a bridge-like connector sheet 41. The connector sheet 41 is symmetric about the center line L and has lateral edge portions 41a opposite to each other in the transverse direction B having respective surfaces 42 facing the garment-contactable sheet 3 permanently bonded to the garment-contactable sheet 3 with adhesives 44. The connector sheet 41 has an intermediate section 41b extending between the lateral edge portions 41a and free from the garment-contactable sheet 3, and a lower surface 43 of the intermediate section 41b opposite to its upper surface 42 is formed symmetrically about the center line L with a pair of pressure-sensitive adhesive regions 45 spaced from each other in the transverse direction B by means of which the lower surface 43 is detachably fastened to the pants as a garment 32 (See Fig. 7). These pressure-sensitive adhesive regions 45 respectively contain pressure-sensitive adhesives 46 serving as fastening means by means of which the pad 1 is temporarily fastened to the garment 32, and the pressure-sensitive adhesives 46 are protectively covered with a release sheet 47 extending in the transverse direction B in Fig. 7. The positions at which the lateral edge portions 41a of the connector sheet 41 are bonded to the garment-contactable sheet 3 are previously selected so that the intermediate section 41b of the connector sheet 41 may be positioned above the peripheral region 6 of the pad 1 in the height direction C when the pad 1 is erected in the inverted V-shape as shown in Fig. 7. Preferably the lateral edge portions 41a of the connector sheet 41 are bonded to the garment-contactable sheet 3 above the peripheral region 6 as shown by way of example in Fig. 7. The pressure-sensitive adhesive regions 45 in the connector sheet 41 lie inside the peripheral region 6 of the pad 1 in the transverse direction B and above the peripheral region 6 in the height direction C. As will be apparent from Fig. 8, the rear endportion R of the pad 1 has the same configuration as the rear end portion R of the pad 1 shown in Fig. 2.

In the pad 1 constructed in the manner as has been described above, the connector sheet 41 functions in the same fashion as that of the adhesives 11 shown in Fig. 2 to prevent the inverted V-shaped pad 1 from excessively expanding in the transverse direction B and thereby to maintain the pad 1 in the properly bent posture. To put the pad 1 on the wearer's body, the finger 51 indicated by an imaginary line in Fig. 7 may be inserted from the end of the rear end portion R of the pad 1 into a space defined between the respective halves of the garment-contactable sheet 3 facing to each other so that the finger 51 may be inserted into a space 52 defined between the garment-contactable sheet 3 and the connector sheet 41. In this way, the pad 1 is captured by the finger 51 and easily brought in close alignment with the wearer's anus. The release sheet 47 may be peeled off before the finger 51 is inserted into the space 52 or after the pad 1 has been brought in close alignment with the anus. In any case, the pad 1 should not be unintentionally bonded to the inner surface of the pants 32 even when the peripheral region 6 of the pad 1 may come in contact with the pants 32 in the course of pulling the pants up. This is because the pressure-sensitive adhesives 46 lie inside and above the peripheral region 6 of the pad 1. The posture of the pad 1 fastened to the pants 32 after the pants 32 have been fully pulled up is indicated by imaginary lines in Figs. 7 and 8. As shown, the portion of the pad 1 in the vicinity of the peripheral region 6 is deformed along the inner surface of the pants 32.

Fig. 9 is a partially cutaway plan view of a flat pad 1a according to the bodily fluid absorbent wearing article of Figure 6 obtained, in the same manner as in the case of Fig. 4, by flatly unfolding the skin-contactable sheet 2, the garment-contactable sheet 3 and the core 4 collectively bent along the center line L. The flat pad 1a according to this embodiment has a shape different from the shape as shown by way of example in Fig. 4. The peripheral region 6 includes segments 6a extending in parallel to the center line L, the end of the front end portion F extends outwardly of the flat pad 1a at a sharp angle so as to define a convex end 48, and the end of the rear end portion R extends inwardly of the flat pad 1a at the same angle as the sharp angle of the convex end 48 so as to define a concave end 49. The flat pad 1a shaped in this manner is advantageous from the viewpoint that the quantity of scraps left behind in the course of making the respective members including the garment-contactable sheet 2 can be reduced. It should be noticed that the connector sheet 41 is folded back on itself together with the release sheet 47 and the connector sheet 41 is bonded with adhesives 44 to a section 3a of the garment-contactable sheet 3 lying on one side of the center line L. The garment-contactable sheet 3 is folded back along the center line L and its section 3b lying on the other side of the center line L is laid over the connector sheet 41 and bonded thereto with the adhesives 44. The pad 1 is obtained by folding such flat pad 1a in two.

While the connector sheet 41 in the pad 1 shown in Fig. 6 may be formed of sheet materials such as nonwoven fabrics, woven fabrics, paper or plastic films, the preferred connector sheet 41 is formed of water-disintegrable sheet materials. With regard to the connector sheet 41, the pressure-sensitive adhesives 46 and pair of pressure-sensitive adhesive regions 45 arranged to be spaced from each other in Fig. 7 may be replaced by the pressure-sensitive adhesives 46 coated so that a pair of pressure-sensitive adhesive regions 45 may become contiguous to each other in the transverse direction B.

The pressure-sensitive adhesives 12 in Fig. 2 and the pressure-sensitive adhesives 46 in Fig. 7, both serving as means to fasten the pad 1 to the pants 32, may be replaced by a hook member of a fastener comprised of a hook member and a loop member adapted to be detachably engaged with each other and widely known by the name Magic Tape (trademark) wherein the inner surface of the pants 32 is used as the loop member which is the counterpart of the hook member.

The present invention having been described above by way of example with respect to the hemorrhoid pad 1 is applicable also to other types of bodily fluid absorbent wearing articles, such as disposable labial pads.

### {Reference Signs List}

- 1: bodily fluid absorbent wearing article (hemorrhoid pad)
- 1a: flatly unfolded pad
- 2: skin-contactable sheet
- 3: garment-contactable sheet
- 4: core
- 6: peripheral region
- 11: bonding means (adhesives)
- 12: fastening means (pressure-sensitive adhesives)
- 20: contour line
- 40: convexly curved segments
- 41: bonding means (connector sheet)
- 46: fastening means (pressure-sensitive adhesives)
- A: longitudinal direction
- B: transverse direction
- L: center line

## Claims

1. A disposable bodily fluid absorbent wearing article (1) having a longitudinal direction (A), a transverse direction (B) and a thickness direction being orthogonal one to another, and comprising a liquid pervious skin-contactable sheet (2), a garment-contactable sheet (3) and a bodily fluid absorbent core (4) sandwiched between the skin-contactable sheet,
the garment-contactable sheet layered in the thickness direction so as to form a flat pad (1a) being symmetric about a center line (L) extending in the longitudinal direction and bisecting a dimension of the pad in the transverse direction wherein the flat pad is bent in two halves along the center line with the skin-contactable sheet facing outward so as to define an inverted V-shaped cross section,
the respective halves of the garment-contactable sheet facing each other in the transverse direction about the center line being partially connected to each other by connector means so that the pad may be maintained in a state of being bent in the inverted V-shape, and
the bodily fluid absorbent wearing article being provided with fastening means (46) adapted for detachably fastening the garment-contactable sheet to the wearer's garment, **characterized in that**:
the connector means comprises a connector sheet (41) having both lateral edge portions (41a) in the transverse direction attached to portions of the respective halves of the garment-contactable sheet.

2. The bodily fluid absorbent wearing article defined by claim 1, wherein the skin-contactable sheet and the garment-contactable sheet extend outward beyond a peripheral edge of the core and put flat and bonded together along respective extending portions to define a peripheral region (6) of the bodily fluid absorbent wearing article and the fastening means are formed inside the peripheral region.

3. The bodily fluid absorbent wearing article defined by claim 1, wherein the flat pad has a contour line (20) defining a planar shape thereof and the contour line includes convexly curved segments (40) so as to be furthest from the center line and the flat pad is formed with the fastening means inside the convexly curved segments.

4. The bodily fluid absorbent wearing article defined by any one of claims 1 through 3, wherein the fastening means are formed of one of a pressure-sensitive adhesive and a hook member of a mechanical fastener.

5. The bodily fluid absorbent wearing article defined by any one of claims 1 through 4, wherein the skin-contactable sheet, the garment-contactable sheet and the connector sheet are respectively formed of water-disintegrable sheets and the core is an assembly of liquid-absorbent fibers each having a fiber length of 20mm or less.

6. The bodily fluid absorbent wearing article defined by any one of claims 1 through 5, wherein any one of the skin-contactable sheet, the garment-contactable sheet and the connector sheet is formed of a water-disintegrable nonwoven fabric containing ultrafine thermoplastic fibers each having a fineness in a range of 0.01 to 0.5dtex and a fiber length in a range of 3 to 10mm in an amount of 10 to 50% by mass.

## Patentansprüche

1. Einweg-Kleidungsstück (1) zur Aufnahme von Körperflüssigkeiten, mit einer Längsrichtung (A), einer Querrichtung (B) und einer Dickenrichtung, die jeweils senkrecht zueinander verlaufen, umfassend eine flüssigkeitsdurhlässige, für Hautkontakt bestimmte Lage (2), eine für Kleidungskontakt bestimmte Lage (3) und einen Körperflüssigkeiten aufnehmenden Kern (4) zwischen der für Hautkontakt bestimmten Lage,
wobei die für Kleidungskontakt bestimmte Lage in der Dickenrichtung so geschichtet ist, dass sie ein flaches Kissen (1a) bildet, das symmetrisch um eine Mittellinie (L) angeordnet ist, die sich in der Längsrichtung erstreckt und eine Erstreckung des Kissens in der Querrichtung schneidet, wobei das flache Kissen entlang der Mittellinie in zwei Hälften gebogen ist und die für Hautkontakt bestimmte Lage zur Bildung eines umgekehrt V-förmigen Querschnitts nach außen gekehrt ist,
wobei die jeweiligen Hälften der für Kleidungskontakt bestimmten Lage, die einander in der Querrichtung um die Mittellinie zugekehrt sind, teilweise durch Verbindungsmittel miteinander verbunden sind, so dass das Kissen in einem um die umgekehrte V-Form gebogenen Zustand verbleibt, und,
wobei das Kleidungsstück zur Aufnahme von Körperflüssigkeiten mit Befestigungsmitteln (46) versehen ist, die zur lösbaren Befestigungs der für Kleidungskontakt bestimmten Lage an der Kleidung des Trägers ausgelegt sind, **dadurch gekennzeichnet, dass**:
das Verbindungsmittel eine Verbindungslage (41) umfasst, deren zwei seitliche Randpartien (41 a) in der Querrichtung an Partien der jeweiligen Hälften der für Kleidungskontakt bestimmten Lage befestigt sind.

2. Kleidungsstück zur Aufnahme von Körperflüssigkeiten nach Anspruch 1, wobei sich die für Hautkontakt bestimmte Lage und die für Kleidungskontakt bestimmte Lage nach außen über einen Umfangsrand des Kerns hinaus erstrecken und entlang den jeweiligen ausstreckenden Partien flach gelegt und miteinander verbunden sind, um einen Umfangsbereich (6) des Kleidungsstücks zur Aufnahme von Körperflüssigkeiten zu definieren, und wobei die Befestigungsmittel innerhalb des Umfangsbereichs ausgebildet sind.

3. Kleidungsstück zur Aufnahme von Körperflüssigkeiten nach Anspruch 1, wobei das flache Kissen eine Konturlinie (20) hat, die ihre Flachform definiert, und wobei die Konturlinie konvex gebogene, am weitesten von der Mittellinie entfernte Segmente (40) umfasst, und wobei das flache Kissen mit den Befestigungsmitteln innerhalb der konvex gebogenen Segmente ausgebildet ist.

4. Kleidungsstück zur Aufnahme von Körperflüssigkeiten nach einem der Ansprüche 1 bis 3, wobei die Befestigungsmittel aus einem Haftklebstoff oder einem Hakenglied eines mechanischen Befestigungselements bestehen.

5. Kleidungsstück zur Aufnahme von Körperflüssigkeiten nach einem der Ansprüche 1 bis 4, wobei die für Hautkontakt bestimmte Lage, die für Kleidungskontakt bestimmte Lage und die Verbindungslage jeweils von in Wasser zersetzbaren Lagen gebildet sind und der Kern eine Einheit aus Flüssigkeit aufnehmenden Fasern, jeweils mit einer Faserlänge von höchstens 20 mm, ist.

6. Kleidungsstück zur Aufnahme von Körperflüssigkeiten nach einem der Ansprüche 1 bis 5, wobei wobei die für Hautkontakt bestimmte Lage, die für Kleidungskontakt bestimmte Lage und die Verbindungslage jeweils von in Wasser zersetzbarem Faservlies gebildet sind, das ultrafeine thermoplastische Fasern mit einer Feinheit in einem Bereich von 0,01 bis 0,5 Dezitex und einer Faserlänge in einem Bereich von 3 bis 10 mm in einer Menge von 10 bis 50 Massen-% enthält.

## Revendications

1. Article à porter jetable absorbant un fluide corporel (1) ayant une direction longitudinale (A), une direction transversale (B) et une direction d'épaisseur étant orthogonales les unes par rapport aux autres, et comprenant une feuille perméable aux fluides pouvant être en contact avec la peau (2), une feuille pouvant être en contact avec le vêtement (3) et un coeur absorbant un fluide corporel (4) intercalé entre la feuille pouvant être en contact avec la peau,
la feuille pouvant être en contact avec le vêtement disposé dans la direction d'épaisseur de manière à former un tampon plat (1a) étant symétrique le long d'une ligne centrale (L) s'étendant dans la direction longitudinale et coupant en deux une dimension du tampon dans la direction transversale dans laquelle le tampon plat est plié en deux moitiés le long de la ligne centrale avec la feuille pouvant être en contact avec la peau orientée vers l'extérieur de manière à définir une section transversale en forme de V renversé,
les moitiés respectives de la feuille pouvant être en contact avec le vêtement se faisant face dans la direction transversale autour de la ligne centrale étant partiellement connectées l'une à l'autre par des moyens de connexion de sorte que le tampon peut être maintenu dans un état plié dans la forme de V renversé, et
l'article à porter jetable absorbant un fluide corporel étant pourvu de moyens de d'attache (46) adaptés pour attacher de manière amovible la feuille pouvant être en contact avec le vêtement au vêtement de l'utilisateur, **caractérisé en ce que** :
le moyen de connexion comprend une feuille de connexion (41) ayant les deux parties de bord latérales (41a) dans la direction transversale attachées aux parties des moitiés respectives de la feuille pouvant être en contact avec le vêtement.

2. Article à porter jetable absorbant un fluide corporel selon la revendication 1, dans lequel la feuille pouvant être en contact avec la peau et la feuille pouvant être en contact avec le vêtement s'étendent vers l'extérieur au-delà d'un bord périphérique du coeur et mises à plat et liées ensemble le long des parties respectives s'étendant vers l'extérieur pour définir une région périphérique (6) de l'article à porter jetable absorbant un fluide corporel et les moyens d'attache sont formés à l'intérieur de la région périphérique.

3. Article à porter jetable absorbant un fluide corporel selon la revendication 1, dans lequel le tampon plat a une ligne de contour (20) définissant une forme planaire de celui-ci et la ligne de contour comprend des segments courbés de manière convexe (40) de manière à être la plus éloignée possible de la ligne centrale et le tampon plat est formé avec les moyens d'attache à l'intérieur des segments courbés de manière convexe.

4. Article à porter jetable absorbant un fluide corporel selon l'une quelconque des revendications 1 à 3, dans lequel les moyens d'attache sont formés par un adhésif sensible à la pression et un élément d'agrafe d'une attache mécanique.

5. Article à porter jetable absorbant un fluide corporel selon l'une quelconque des revendications 1 à 4, dans lequel la feuille pouvant être en contact avec la peau, la feuille pouvant être en contact avec le vêtement et la feuille de connexion sont formées respectivement dans des feuilles pouvant se désintégrer dans l'eau et le coeur est un assemblage de fibres absorbant un fluide ayant chacune une longueur de fibre de 20 mm ou moins.

6. Article à porter jetable absorbant un fluide corporel selon l'une quelconque des revendications 1 à 5, dans lequel l'une quelconque de la feuille pouvant être en contact avec la peau, la feuille pouvant être en contact avec le vêtement et la feuille de connexion est formée d'une étoffe non tissée pouvant se désintégrer dans l'eau contenant des fibres thermoplastiques ultrafines ayant chacune une finesse comprise dans un intervalle de 0,01 à 0,5 dtex et une longueur de fibre comprise dans un intervalle de 3 à 10 mm dans une quantité de 10 à 50 % en masse.
